# EUROPEAN PATENT APPLICATION

(11) **EP 2 937 699 A1**
(43) Date of publication of application: **28.10.2015**
(21) Application number: 13865542.8
(22) Date of filing: 20.12.2013
(51) Int. Cl.: G01N 33/86, G01N 33/49, G01N 37/00

(54) **METHOD FOR COMPREHENSIVE ASSESSMENT OF PLATELET AGGREGATION**

(30) Priority: 20.12.2012 JP 2012278452
(71) Applicant: FUJIMORI KOGYO CO., LTD., Tokyo 160-0023 (JP)
(72) Inventor: HOSOKAWA, Kazuya, Tokyo 160-0023 (JP); WADA, Tomoko, Tokyo 160-0023 (JP); HASEGAWA, Takaaki, Tokyo 160-0023 (JP)
(74) Representative: Jones, Nicholas Andrew
(86) International application number: PCT/JP2013/084369
(87) International publication number: WO 2014/098242

(57) **Abstract**

A method for comprehensively and quantitatively analyzing platelet aggregability, and stability and persistence of platelet aggregates, which method comprises the steps of: reacting a platelet-activating reagent with blood in a closed container; injecting a liquid which is not mixable with blood into the container using a pump connected to a first end of the container, thereby pushing the mixture of the platelet-activating reagent and the blood out from the container and allowing the mixture to pass through a filter in a filter device connected to a second end of the container; and measuring the pressure exerted on the pump.

## Description

### TECHNICAL FIELD

The present invention relates to a method and a test apparatus for investigating platelet aggregability, more specifically, a method in which blood mixed with a platelet-activating reagent is pushed out from a container using a pump to allow the mixture to pass through a filter, and the integrated value of the pressure change during this process is used for comprehensive evaluation of platelet aggregability, and stability and persistence of aggregates formed, and an apparatus to be used for the method.

### BACKGROUND ART

### (Problems in Prior Art for Platelet Aggregation Test)

Activation and aggregation of platelets play a central function in formation of a thrombus (white thrombus) in an artery, or in primary hemostasis.

When injury of a blood vessel occurred, platelets directly or indirectly bind to collagen present under vascular endothelial cells. Under a gentle flow of blood (under low shearing stress), direct binding by collagen receptors such as GPVI mainly occurs. Under a rapid flow of blood (under high shearing stress), vWF binds to collagen, and GPIbα receptors of platelets then bind to the vWF, indirectly causing binding of the platelets to the collagen. The direct or indirect interaction with collagen activates platelets, and this stimulation causes release of various platelet-activating substances such as adenosine diphosphate (ADP) and serotonin from dense bodies and α granules.

The released platelet-activating factors activate the platelets from which those factors were released, and platelets in the vicinity thereof. In the activated platelets, the structural change of a fibrinogen receptor GPIIbIIIa into the activated form occurs to give the platelets high affinity to fibrinogen. The activated platelets are successively cross-linked through dimeric fibrinogen to form platelet aggregates.

Conventionally, the light transmission method is mainly used for measurement of platelet aggregability. In this method, a platelet-activating reagent is mixed with platelet rich plasma (hereinafter referred to as PRP) separated from blood, and an aggregation rate curve is prepared based on changes (decreases) in the turbidity with time due to platelet aggregation (Patent Document 1).

Since preparation of PRP from blood is laborious, platelet aggregation measurement devices applicable to whole-blood measurement have been devised for simpler measurement of platelet aggregation. Major examples of the devices include those in which whole blood is mixed with a platelet activation substance, and changes in the electric resistance due to adhesion/aggregation of platelets to an electrode immersed in the blood are measured (Non-patent Documents 1 and 2).

Other reported examples include methods in which mixtures of blood and a plurality of concentrations of a platelet-activating reagent are left to stand to allow the reaction to proceed for several minutes, and each mixture is then sucked to allow the mixture to pass through a mesh, thereby determining the threshold of the platelet-activating reagent based on the suction pressure and judging whether the platelet aggregability is normal or not (Patent Document 2, Patent Document 3). This method is a method for measuring the whole blood platelet aggregability in which a threshold coefficient for platelet aggregation in whole blood is calculated from an aggregation curve and the aggregation threshold, and whether the platelet aggregability in whole blood is normal or not is judged based on the area where the platelet aggregation threshold coefficient is positioned.

Patent Document 4 discloses a platelet function test method in which anticoagulated blood mixed with a weak platelet-activating reagent is allowed to pass through a capillary having a platelet adhesion-promoting layer on at least a part of the inner surface, and the behavior of the blood in the capillary is observed or measured to test the platelet function.

### PRIOR ART DOCUMENTS

### [Patent Documents]

Patent Document 1: JP 8-10226 B
Patent Document 2: JP 2005-291849 A
Patent Document 3: JP 2006-300859 A
Patent Document 4: WO 2010/018833

### [Non-patent Documents]

Non-patent Document 1: Morphologic alterations of blood cells in the impedance aggregometer. Blood Cells. 1985; 11(2): 325-36, 337-9.
Non-patent Document 2: A method of testing platelet aggregation in native whole blood. Thromb Res. 1985 Apr 1; 38(1): 91-100.

### SUMMARY OF THE INVENTION

In conventional measurement of platelet aggregability, formation of aggregates due to addition of a platelet-activating reagent and the threshold of its occurrence can be measured. However, quantitative measurement of the stability and persistence of the aggregates is difficult. That is, the threshold concentration of a platelet-activating reagent at which clogging occurs can be determined by the methods in which blood is allowed to react with different concentrations of a platelet-activating reagent for several minutes, and then sucked and allowed to pass through a filter while negative pressure due to clogging of the filter is detected for measuring the concentration at which platelet aggregation occurs, but, since the flow rate of the passing blood changes depending on the degree of clogging of the mesh, its accurate and quantitative evaluation is impossible.

Moreover, in cases where the suction is carried out, the presence of air between the blood and the suction syringe prevents maintenance of a constant flow rate especially in the low flow rate region (not more than 100 µm/minute), and accurate measurement of the pressure changes with time due to passage of the blood through the filter at a constant flow rate is impossible. There is also a problem that the platelet-activating reagent needs to be prepared at various concentrations and sucked, which is laborious.

The present invention was made under the above-described circumstances, and aims to provide a method and apparatus which enable quantitative evaluation of the rate of initiation of platelet aggregation, and stability and persistence of platelet aggregates.

In order to solve the problems described above, the present invention provides a method for analyzing platelet aggregability and stability of platelet aggregates, the method comprising the steps of: reacting a platelet-activating reagent with blood in a closed container; injecting a liquid which is not mixable with blood into the container using a pump connected to a first end of the container, thereby pushing the mixture of the platelet-activating reagent and the blood out from a second end of the container and allowing the mixture to pass through a filter in a filter device connected to the second end of the container; and measuring the pressure exerted on the pump.

The liquid which is not mixable with blood is preferably mineral oil.

The platelet-activating reagent is preferably ADP at a final concentration of 1 to 10 µM, collagen at a final concentration of 0.5 to 10 µg/ml, or arachidonic acid at a final concentration of 0.2 to 20 mM.

The injection rate of the liquid which is not mixable with blood is preferably 5 to 200 µl/minute. Since 25 µl to 1 ml of blood is enough for carrying out the reaction for 5 minutes, the measurement is possible with a proper amount of blood sample.

The filter preferably has a mesh having a pitch size or diameter of 10 µm to 50 µm.

The liquid which is not mixable with blood is preferably injected to the container such that the mixture of the platelet-activating reagent and the blood reaches the filter 20 seconds to 2 minutes after the mixing of the platelet-activating reagent with the blood.

The present invention also provides an apparatus for analyzing platelet aggregability and stability of platelet aggregates, the apparatus comprising a closed container, a pump connected to a first end of the container, a sensor for measuring the pressure exerted on the pump, and a filter device air-tightly connected to a second end of the container. The closed container is preferably composed of a blood storage section and a cap for tightly sealing the blood storage section; the closed container is preferably connected to a filter device through the cap; the filter device preferably comprises a filter section and a waste liquid storage section; and an air hole(s) is/are preferably present in the waste liquid storage section

By the method and apparatus of the present invention, stability and persistence of platelet aggregation can be quantitatively evaluated.

The liquid which is not mixable with blood is preferably mineral oil since mineral oil can efficiently push the blood out from the container and allow the blood to reach the filter device.

The platelet-activating reagent is preferably ADP at a final concentration of 1 to 10 µM, collagen at a final concentration of 0.5 to 10 µg/ml, or arachidonic acid at a final concentration of 0.2 to 20 mM from the viewpoint of easily allowing formation of platelet aggregates and efficiently obtaining a pressure rise waveform.

The injection rate of the liquid which is not mixable with blood is preferably 5 to 200 µl/minute since, at this injection rate, accurate liquid transfer is possible, and the measurement can be continued for a certain period of time even with a small amount of blood.

The pitch size or diameter of the mesh of the filter is preferably 10 µm to 50 µm from the viewpoint of obtaining a highly reproducible pressure rise waveform due to platelet aggregates.

The liquid which is not mixable with blood is preferably injected to the container such that the mixture of the platelet-activating reagent and the blood reaches the filter 20 seconds to 2 minutes after the mixing of the platelet-activating reagent with the blood since, in this case, the platelets pass through the filter while being activated, and therefore the initial rise of the pressure waveform tends to reflect the rate of formation of the aggregates.

In cases where the mixture is allowed to pass through the filter quickly after the initiation of the platelet aggregability reaction but before its completion, the integrated value of the pressure waveform comprehensively reflects the formation rate, stability, and persistence of platelet aggregates.

### BRIEF DESCRIPTION OF THE DRAWINGS

Fig. 1 is a diagram illustrating the first embodiment of the platelet aggregability measurement apparatus of the present invention.
Fig. 2 is a graph showing the result of measurement of the pressure using the platelet aggregability measurement apparatus of the present invention (Example 1).
Fig. 3 is a graph showing the result of measurement of the pressure using the platelet aggregability measurement apparatus of the present invention (Example 2).
Fig. 4 is a graph showing the result of measurement of the pressure using the platelet aggregability measurement apparatus of the present invention (Example 3).
Fig. 5 is a graph showing the result of measurement of the pressure using the platelet aggregability measurement apparatus of the present invention (Example 4).
Fig. 6 is a graph showing the result of measurement of the pressure using the platelet aggregability measurement apparatus of the present invention (Example 5).
Fig. 7 is a graph showing the result of measurement of the pressure using the platelet aggregability measurement apparatus of the present invention (Example 6).
Fig. 8 is a diagram illustrating the second embodiment of the platelet aggregability measurement apparatus of the present invention.
Fig. 9 is a diagram illustrating an example of the filter in the platelet aggregability measurement apparatus of the present invention.
Fig. 10 is a diagram showing the third embodiment of the platelet aggregability measurement apparatus of the present invention (a blood storage container and a filter device).
Fig. 11 is a graph showing the result of measurement of the pressure using the platelet aggregability measurement apparatus of the present invention (Example 7).
Fig. 12 is a graph showing the result of measurement of the pressure using the platelet aggregability measurement apparatus of the present invention (Example 8).

### EMBODIMENTS FOR CARRYING OUT THE INVENTION

In the present invention, a platelet-activating reagent is mixed with blood in a container air-tightly connected with a pump for transferring a liquid which is not mixable with blood, such as mineral oil. The liquid is then injected into the container at a constant flow rate to allow the mixture of the platelet-activating reagent and the blood to flow at a constant flow rate into a filter in a filter device connected to another end of the container, while the waveform indicating the pressure change during this process is measured with time to measure the platelet aggregability and the stability of platelet aggregates, and to carry out their comprehensive evaluation.

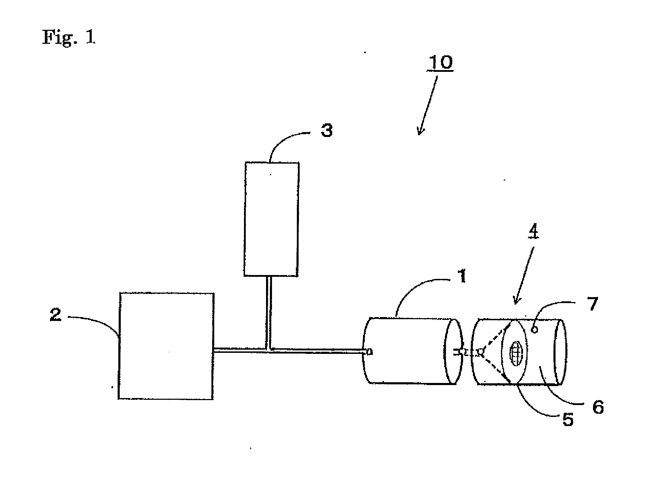

First, the method for measuring the platelet aggregability and the apparatus therefor of the present invention are described with reference to figures. In the present invention, "blood" includes both whole blood and platelet-rich plasma. Fig. 1 is a schematic diagram illustrating one embodiment of the platelet aggregability measurement apparatus of the present invention. However, the apparatus of the present invention is not limited to this embodiment.

The invention is described below based on Fig. 1.

An apparatus 10 according to the first embodiment of the present invention comprises a blood storage container 1 (which may be hereinafter simply referred to as container 1), a liquid transfer pump 2 air-tightly connected to a first end of the container 1, a pressure sensor 3 for measuring the pressure exerted on the pump 2, and a filter device 4 connected to a second end of the container 1.

The filter device 4 comprises a filter section 5 and a waste liquid storage section 6.

During the measurement using the filter device 4, its opening (penetrating hole) communicating with the filter section may be connected with a projection at the second end of the container 1 in which an opening is formed.

The filter device 4 can be prepared by, for example, installing the filter section 5 having a filter at the center of a circle, in a cylindrical container such that the outer circumference of the filter is in intimate contact with the container. By this, the blood sample that has passed through the filter can be stored in the waste liquid storage section 6 as a waste liquid. There is an air hole 7 in the waste liquid storage section 6, and this allows aeration and therefore enables accurate pressure measurement.

Examples of the material of the container 1 include metals, glasses, plastics, and silicones. The material is preferably transparent. In order to suppress blood coagulation at unexpected sites, the inside of the container may be treated with PDMS (polydimethylsiloxane) or poly 2-methoxyethylacrylate (PMEA).

First, in the method of the present invention, blood is reacted with a platelet-activating reagent in the container 1.

The blood stored in the container 1 is preferably anticoagulated blood.

Examples of the anticoagulant herein include sodium or potassium citrate, sodium or potassium oxalate, ACD (Acid Citrate Dextrose), and salts of ethylenediaminetetraacetic acid (EDTA). Such anticoagulants may be used as powders, freeze-dried products, or solutions such as aqueous solutions. Among these anticoagulants, 3.2% sodium citrate is preferred since it is commonly used and easily available. In such a case, 1 volume of the anticoagulant is preferably used for 9 volumes of blood.

Other examples of anticoagulants which may be used include heparin, hirudin, thrombin inhibitors, and maize-derived trypsin inhibitors (1977. J. Biol. Chem 252. 8105). A plurality of anticoagulants may be used.

Examples of the method for obtaining the anticoagulated blood include a method in which blood is collected using a syringe or vacuum blood collection tube in which the anticoagulant is preliminarily placed, and a method in which the anticoagulant is quickly added to blood immediately after collection.

Examples of the platelet-activating reagent to be mixed with the anticoagulated blood include ADP, collagen, arachidonic acid, and ristocetin. The platelet-activating reagent is used at a concentration which causes platelet activation in healthy individuals. The concentration which causes platelet activation is, for example, a final concentration of 1 to 10 µM in cases of ADP; a final concentration of 0.5 to 10 µg/ml in cases of collagen; and a final concentration of 0.2 to 20 mM in cases of arachidonic acid.

The blood may be mixed with the platelet-activating reagent in advance, and the resulting mixture may then be placed in the container 1. However, it is preferred to preliminarily place the platelet-activating reagent in the dry state or liquid state in the container 1, followed by adding the blood thereto to allow the reaction.

For example, the end of the container 1 to which the filter device is to be connected may be sealed with a cap through which a needle can penetrate, which cap is made of a material such as rubber, and blood collected with a syringe may be injected into the container to allow the blood to react with the platelet-activating reagent preliminarily placed in the container 1.

The liquid which is not mixable with blood in the liquid transfer pump 2, air-tightly connected to the first end of the container 1 through a tube, is injected into the container 1 using the pump 2, and, by this, the mixture of the platelet-activating reagent and blood in the container 1 is pushed out into the filter device 4 connected to the second end of the container 1.

In terms of the timing of injecting the liquid which is not mixable with blood into the container 1 to push the mixture of the platelet-activating reagent and blood out into the filter section, the injection is preferably begun such that the mixture of the platelet-activating reagent and blood reaches the filter 20 seconds to 2 minutes (preferably 20 seconds to 1 minute) after the mixing of the blood with the platelet-activating reagent.

For example, in Patent Document 2, blood is mixed with 0.5, 1, 2, or 4 µM ADP, and the resulting mixture is allowed to react for 5 minutes, followed by sucking the mixture through a filter in order to draw an aggregation curve based on the suction pressure due to occurrence of clogging and to thereby determine the threshold concentration of the platelet-activating substance at which platelet aggregation occurs.

However, in this method, the platelet-activating reagent needs to be prepared at different concentrations, which is laborious, and, although determination of the concentration threshold at which the aggregation occurs is possible, the results do not reflect the stability, persistence, and the like of the platelet aggregates formed.

On the other hand, in the present invention, blood mixed with a platelet-activating reagent within the concentration range in which platelet aggregation occurs in healthy individuals is allowed to pass through a filter 20 seconds to 2 minutes (preferably 20 seconds to 1 minute) after the mixing. The initiation of the pressure increase reflects the degree (rate) of formation of platelet aggregates, and the maximum pressure reflects the stability of the aggregates. The integrated value of the pressure reflects both the aggregate formation and the stability of the aggregates formed.

The liquid in the liquid transfer pump is not limited as long as the liquid is not mixed with the blood when the liquid is injected into the container 1, and as long as the mixture of the blood and the platelet-activating reagent can be pushed out from the container 1 by the liquid. An example of the liquid includes mineral oil. By increasing the flow rate in a stepwise manner using the liquid transfer pump, the shear stress can be increased in a stepwise manner.

The mixture of the platelet-activating reagent and blood pushed out from the container 1 reaches the filter device 4, and passes through the filter while causing clogging of the filter due to platelet aggregation.

The blood that has passed through the filter is stored in the waste liquid storage section 6.

The filter is mesh-shaped, and the pitch size or diameter of the mesh is preferably 10 µm to 50 µm, more preferably 20 µm to 50 µm. The area of the filter is preferably 1 to 100 mm². The thickness of the filter is preferably 10 to 100 µm.

For measurement of the stability of platelets, the flow rate is preferably a constant flow rate of 5 to 200 µl/minute, more preferably a constant flow rate of 10 to 100 µl/minute.

The blood is preferably allowed to pass through the filter for 2 minutes to 10 minutes.

A whole blood sample in a relatively small amount, for example, 500 µl, is sufficient for use in the method of the present invention even in cases where the sample is allowed to pass through the filter with time at a low flow rate of, for example, 50 µl/minute for 10 minutes.

The blood container and the filter are preferably warmed at about 37°C using a heater.

By passing of the blood through the filter at a constant rate, filter clogging occurs due to blood coagulation. The platelet aggregability can be evaluated by sensitively measuring the small pressure change caused by the filter clogging, and calculating the integrated value of the pressure waveform obtained during 2 to 10 minutes.

The starting time of the pressure increase (time required for the start of the increase in the pressure after the beginning of the measurement) is mainly associated with the platelet aggregability and the aggregation rate. On the other hand, the maximum pressure reflects the stability and the strength of the platelet aggregates formed, and the integrated value or area under the curve (AUC) of the pressure waveform plotted against time can be used as the comprehensive index.

By allowing the blood containing activated platelets, in the closed container, to flow into the filter using a micropump, pressure changes during its passing through the filter at a constant rate can be accurately measured with an accuracy of 0.1 kPa.

After the beginning of the pressure increase due to clogging of the filter with platelet aggregates, the stability and the strength of the platelet aggregates can be simultaneously measured by further allowing the blood to flow through the filter at a constant flow rate for a certain period of time (about 2 to 10 minutes) while the measurement of pressure changes is continued.

The method of the present invention enables measurement of the beginning of clogging due to formation of platelet aggregates, and the stability and persistence of the aggregates, which depend on the type and concentration of the platelet-activating reagent employed.

For example, in cases where ADP is employed at a concentration of 5 µM, the beginning of the pressure increase (aggregate formation) occurs earlier than in cases where collagen is employed at a concentration of 1.5 µg/ml, but the pressure becomes constant in 2 to 3 minutes. On the other hand, in cases where collagen is employed at a concentration of 1 µg/ml, the beginning of the pressure increase occurs late, but a continuous pressure increase can be observed for 5 to 6 minutes.

Thus, the beginning of the pressure increase, maximum pressure, and the like are differently influenced depending on the platelet-activating reagent employed and the antiplatelet agent used for its suppression.

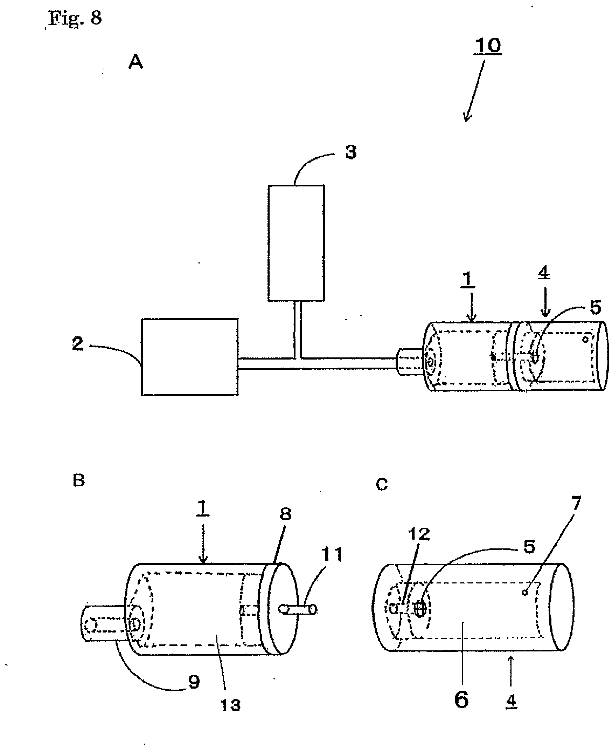

Fig. 8 shows a platelet aggregability measurement apparatus according to the second embodiment of the present invention.

As shown in Fig. 8B, the blood storage container 1 is composed of: a first end 9 in which a penetrating hole is formed, which penetrating hole plays a role as a connecting section for insertion of a tube which connects a liquid transfer pump to the container; a blood storage section 13 for storing blood; a cap 8; and a second end 11 having a projection connected to the cap 8, which projection connects a filter device 4 to the container. After mixing the platelet-activating reagent with blood in the blood storage section, the container is tightly sealed by placing the cap 8, and the filter device 4 is connected to the blood storage container 1.

For elimination of the measurement error, it is preferred to fill the inside of the blood storage section with the mixture of the platelet-activating reagent and blood and then to connect the filter device 4 to the blood storage container 1, followed by allowing the mixture to flow into the filter device. In order to achieve this, first, a closed container may be air-tightly connected to the second end of the blood storage container 1, and the mixture may be discharged in a small amount into the closed container from the blood storage container 1 to fill the blood storage container 1 with the mixture, followed by displacing the closed container and then connecting the blood storage container 1 to the filter device 4.

As shown in Fig. 8C, the filter device 4 has a portion in which a penetrating hole 12 for insertion of the projection of the second end of the blood storage container 1 is formed, a filter 5, and a waste liquid storage section 6. In the waste liquid storage section 6, an air hole 7 is provided.

The filter 5 is placed such that the filter covers the portion corresponding to the penetrating hole 12 in the side in which the waste liquid storage section 6 is connected to the blood storage container 1, and this allows the mixture of the blood and platelet-activating reagent, which has flowed from the blood storage container 1 through the penetrating hole 12, to pass through the filter 5.

As shown in Fig. 8A, during the operation, a tube for connection to the liquid transfer pump 2 is inserted in the first end 9 of the blood storage container 1, and the projection of the second end 11 provided outside the cap 8 of the blood storage container 1 is inserted in the penetrating hole 12 of the filter device 4. Blood mixed with the platelet-activating reagent in the blood storage container 1 is pushed out by the liquid transfer pump 2 into the filter device 4, in which the blood passes through the filter 5, and is then stored in the waste liquid storage section 6.

By passing of the blood through the filter at a constant rate, filter clogging occurs due to blood coagulation. The platelet aggregability can be evaluated by sensitively measuring the small pressure change caused by the filter clogging, and calculating the integrated value of the pressure waveform obtained during 2 to 10 minutes.

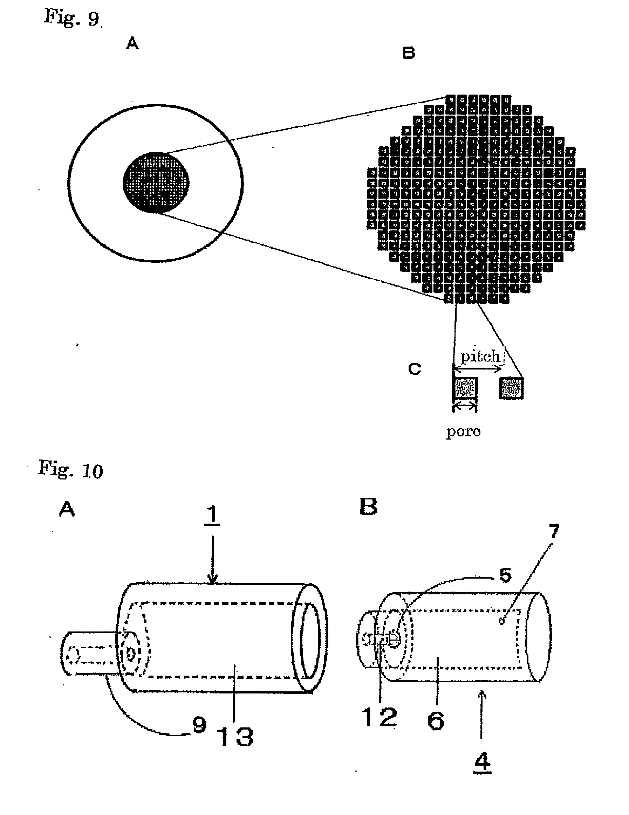

Fig. 9 shows an example of the structure of the filter 5. As shown in the general view in Fig. 9A, a filter is placed at the center such that the filter covers the portion through which the mixture of the platelet-activating reagent and blood pushed out from the container 1 passes. An enlarged view of the filter section is shown in Fig. 9B, and an enlarged view of its openings is shown in Fig. 9C.

A platelet aggregability measurement apparatus according to the third embodiment of the present invention is shown in Fig. 10. Fig. 10A shows a blood storage container 1, and Fig. 10B shows a filter device 4. Unlike the blood storage container of the second embodiment in Fig. 8B, the blood storage container 1 does not have a cap in the second-end side. On the other hand, the filter device has an end which is air-tightly connected to the second end of the blood storage container 1. That is, the platelet-activating reagent is mixed with blood in the blood storage section 13, and the second end of the blood storage container 1 is directly and air-tightly connected to the end of the filter device 4. Thereafter, the mixture passes through the penetrating hole 12 of the filter device 4 and then through the filter, followed by being stored in the waste liquid storage section 6.

### EXAMPLES

The present invention is described below in more detail by way of concrete Examples. However, the present invention is not limited to the Examples.

The apparatus in Fig. 1 was prepared for use in the following experiments.

As the container 1 (blood reservoir), a cylindrical acrylic container having a capacity of 450 µl (inner diameter, 6 mm; depth, 16 mm) was used.

As the filter, a circular nickel micromesh filter having 30 µm × 30 µm square openings (pitch size, 45 µm) and a diameter of 1 mm, placed at the center of the filter section was used.

### Example 1

To the blood reservoir, 13 µl of 200 mM ADP reagent (Dynabite GmbH, Germany) (final concentration, 5.6 µM) was added, and 450 µl of blood collected in a Terumo blood collection tube (Venoject, containing 3.13% sodium citrate) was added thereto. The resulting mixture was mixed in the blood reservoir, and the filter device was connected to the blood reservoir. One minute after the mixing of the blood with the ADP reagent, mineral oil was injected into the blood reservoir at a flow rate of 60 µl/minute to inject the blood into the filter device.

The back pressure exerted on the mineral oil was continuously monitored for 5 minutes at intervals of 1 second using the pressure sensor. In addition, the same measurement was carried out for blood to which AR-C66096 (platelet P2Y₁₂ receptor inhibitor; Tocris Bioscience, UK) was added to a final concentration of 25, 50, 100, or 250 nM.

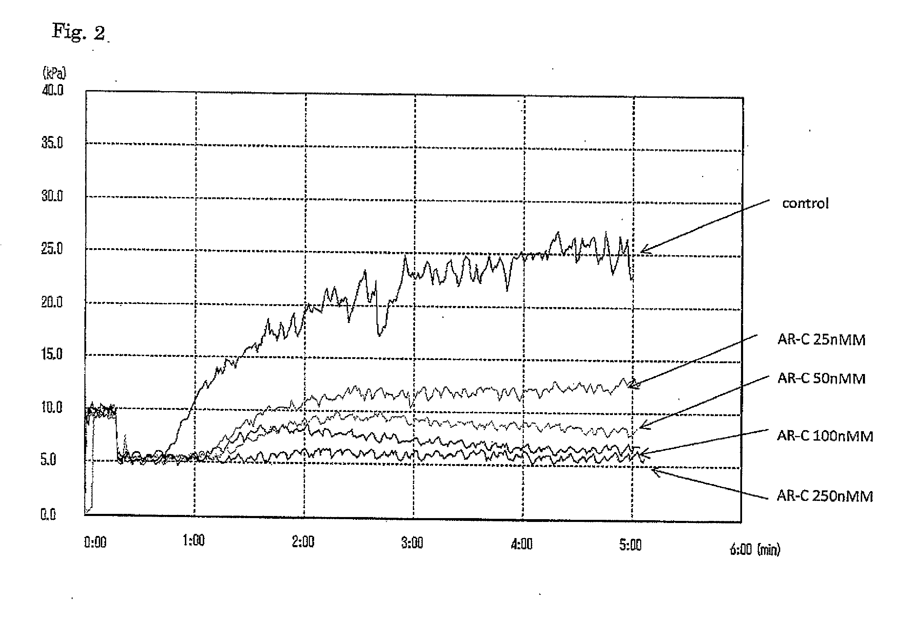

The resulting pressure waveforms were as shown in Fig. 2.

AR-C66096 delayed the beginning of the pressure increase, and suppressed the pressure increase in a concentration-dependent manner. In particular, archshaped pressure curves were drawn in the cases where AR-C66096 was present at 50 nM or 100 nM. These results indicate that the stability and persistence of platelet aggregates were suppressed by the presence of AR-C66096. As shown in Table 1, the area under the curve was suppressed in a manner dependent on the concentration of AR-C66096. From these results, it can be seen that the method of the present invention reflects both the delay of the beginning of the pressure increase and the stability of the pressure, and that quantitative evaluation of platelet aggregation is possible by the method.

**[Table 1]**

| | AR-C66096 | | | |
|---|---|---|---|---|
| Control | 25nM | 50nM | 100nM | 250nM |
| 17.55 | 11.75 | 8.7 | 8.05 | 7.95 |

To provide controls, whole blood platelet aggregation was similarly measured for blood to which AR-C66096 was added to a final concentration of 25, 50, 100, or 250 nM, using Multiplate (impedance-based platelet aggregability analyzer, Dynabite GmbH). As the platelet-activating reagent, ADP (final concentration, 6.5 µM) was used. The AUC values were as shown in Table 2. As a result, no concentration dependence was found, and, in particular, the AUC values observed in the presence of high concentrations of AR-C66096 were less likely to reflect the effect of the high concentrations of AR-C66096. All the impedance curves showed a continuous rise in the value. That is, in the absence of a physical load by blood flow or the like, platelet aggregates that have once adhered/aggregated to the electrode were maintained without breakdown, leading to the increase in the electric resistance.

**[Table 2]**

| | AR-C66096 | | | |
|---|---|---|---|---|
| Control | 25nM | 50nM | 100nM | 250nM |
| 54 | 23 | 19 | 15 | 17 |

### Example 2

To the blood reservoir, 13 µl of 25 µg/ml (final concentration, 0.7 µg/ml) collagen reagent (manufactured by Dynabite GmbH) was added, and 450 µl of blood collected in a Terumo blood collection tube (Venoject, containing 3.13% sodium citrate) was added thereto. After mixing the resulting mixture, the filter device was connected to the blood reservoir. One minute after the mixing of the blood with the collagen reagent, mineral oil was injected into the blood reservoir at a flow rate of 60 µl/minute to inject the blood into the filter device.

The back pressure exerted on the mineral oil was continuously monitored for 5 minutes at intervals of 1 second using the pressure sensor. In addition, the same measurement was carried out for blood to which aspirin was added to a final concentration of 100 µM, or blood to which both 50 µM aspirin and 250 µM ARC66096 were added.

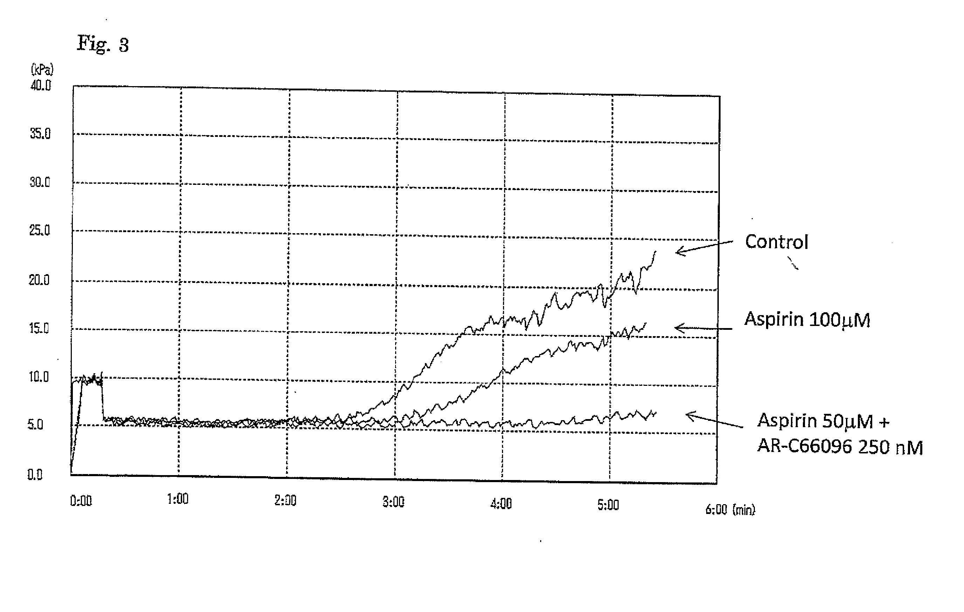

The resulting pressure waveforms were as shown in Fig. 3.

Aspirin delayed the beginning of the pressure increase, and suppressed the pressure increase. The use of the combination of aspirin and AR-C66096 resulted in a synergistic suppression of the pressure increase.

To provide controls, whole blood platelet aggregation was similarly measured for blood to which aspirin was added to a final concentration of 100 µM, and blood to which both 50 µM aspirin and 250 µM AR-C66096 were added, using Multiplate (impedance-based platelet aggregability analyzer, Dynabite GmbH). As the platelet-activating reagent, collagen (final concentration, 3.2 µg/ml) was used. The AUC values of the electric resistance were as shown in Table 3. As a result, an effect of aspirin could be found, but no synergistic was found for aspirin and ARC66096.

**[Table 3]**

| Control | aspirin 100 *µ*M | aspirin 50 *µ*M +AR-C 250 *µ*M |
|---|---|---|
| 38 | 28 | 24 |

### Example 3

To the blood reservoir, 13 µl of 50 µg/ml (final concentration, 1.4 µg/ml) collagen reagent (manufactured by Dynabite GmbH) was added, and 450 µl of blood collected in a Terumo blood collection tube (Venoject, containing 3.13% sodium citrate) was added thereto. After mixing the resulting mixture, the filter device was connected to the blood reservoir. One minute after the mixing of the blood with the collagen reagent, mineral oil was injected into the blood reservoir at a flow rate of 60 µl/minute to inject the blood into the filter device.

The back pressure exerted on the mineral oil was continuously monitored for 5 minutes at intervals of 1 second using the pressure sensor. The measurement was repeated 5 times.

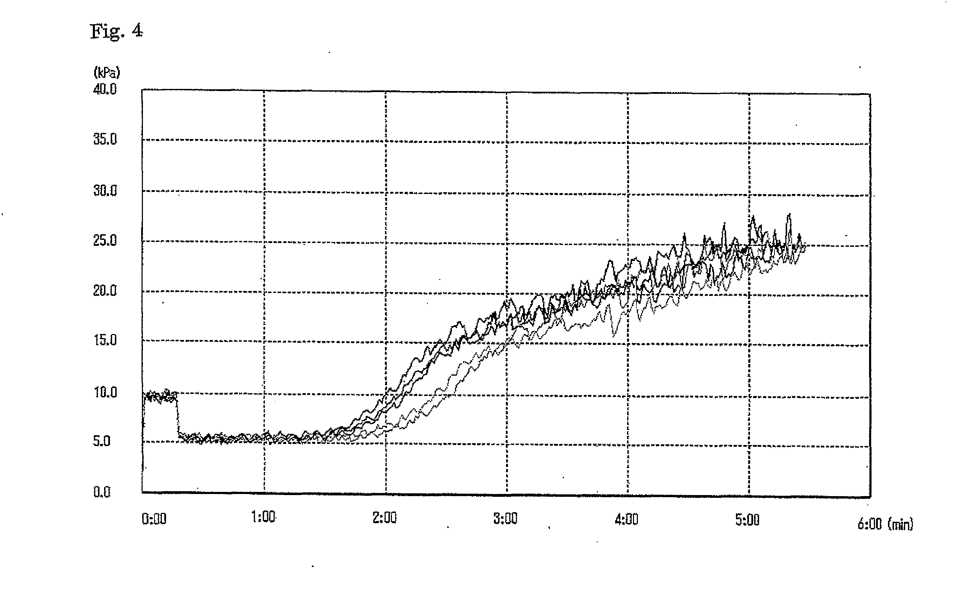

The resulting pressure waveforms were as shown in Fig. 4. The area under the curve as determined by the 5 times of measurement was 15.11±0.8 (mean±SD), and the CV value was 5.3%. Thus, the results were highly reproducible.

### Example 4

To the blood reservoir, 30 or 50 µl (final concentration, 6.25 mM or 10 mM, respectively) of 100 mM arachidonic acid (manufactured by Dynabite GmbH) was added, and 450 µl of blood collected in a Terumo blood collection tube (Venoject, containing 3.13% sodium citrate) was added thereto. After mixing the resulting mixture, the filter device was connected to the blood reservoir. One minute after the mixing of the blood with the collagen reagent, mineral oil was injected into the blood reservoir at a flow rate of 60 µl/minute to inject the blood into the filter device.

The back pressure exerted on the mineral oil was continuously monitored for 5 minutes at intervals of 1 second using the pressure sensor. In addition, the same measurement was carried out for a mixture prepared by mixing 50 µl of an arachidonic acid reagent with blood to which aspirin was added to a final concentration of 100 µM.

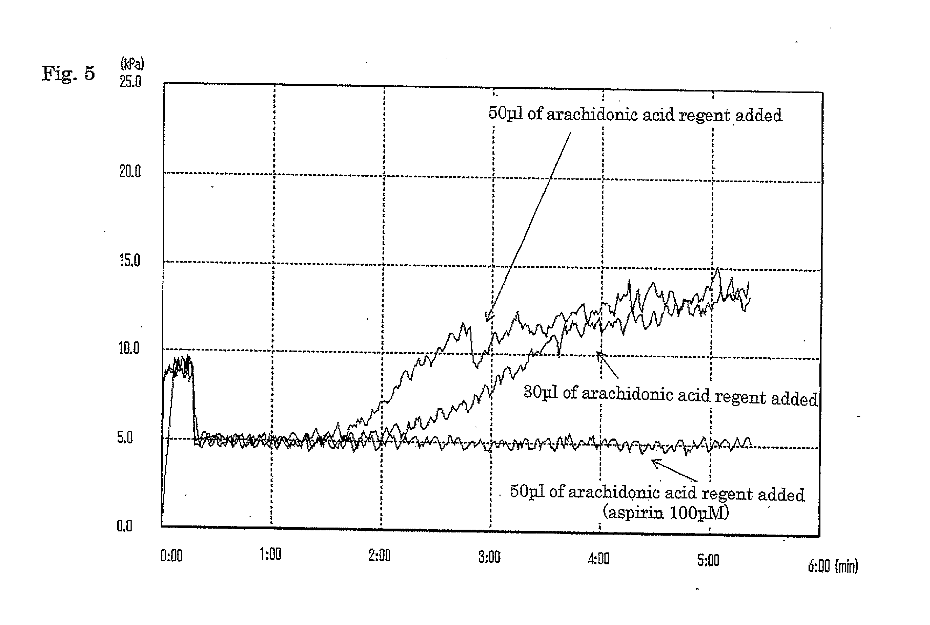

The results on the pressure waveform were shown in Fig. 5. As a result, arachidonic acid activated platelets to increase the pressure, but the pressure increase was suppressed by aspirin.

### Example 5

To the blood reservoir, 20 µl of 1 mM PAR1-activating reagent (peptide sequence, SLFFRN; manufactured by Dynabite GmbH) was added, and 450 µl of blood collected in a Terumo blood collection tube (Venoject, containing 3.13% sodium citrate) was added thereto. After mixing the resulting mixture, the filter device was connected to the blood reservoir. One minute after the mixing of the blood with the collagen reagent, mineral oil was injected into the blood reservoir at a flow rate of 60 µl/minute to inject the blood into the filter device.

The back pressure exerted on the mineral oil was continuously monitored for 5 minutes at intervals of 1 second using the pressure sensor. The same measurement was carried out for blood to which aspirin was added to a final concentration of 100 µM.

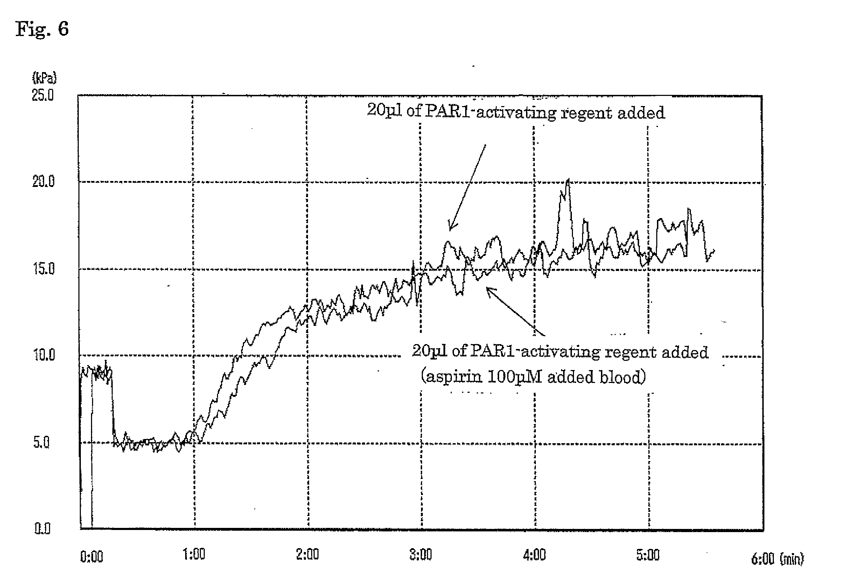

The resulting pressure waveforms were as shown in Fig. 6. The waveforms were relatively similar to the waveform in the case of ADP aggregation. The aggregation waveform obtained by the PAR1-activating peptide was not inhibited by aspirin.

### Example 6

To the blood reservoir, 20 µl of 20 mM PAR4-activating reagent (peptide sequence, AYPGKF; manufactured by Dynabite GmbH) was added, and 450 µl of blood collected in a Terumo blood collection tube (Venoject, containing 3.13% sodium citrate) was added thereto. After mixing the resulting mixture, the filter device was connected to the blood reservoir. One minute after the mixing of the blood with the collagen reagent, mineral oil was injected into the blood reservoir at a flow rate of 60 µl/minute to inject the blood into the filter device.

The back pressure exerted on the mineral oil was continuously monitored for 5 minutes at intervals of 1 second using the pressure sensor. The same measurement was carried out for blood to which aspirin was added to a final concentration of 100 µM.

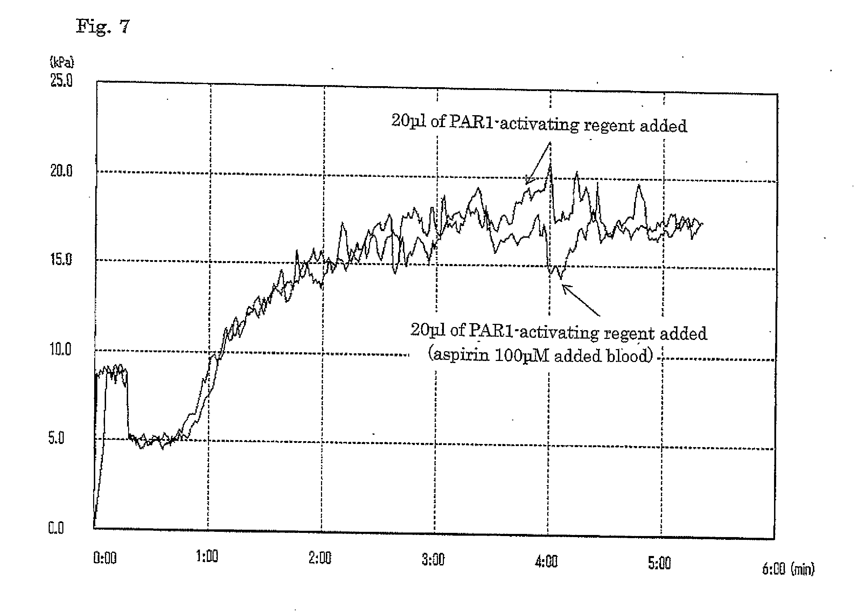

The resulting pressure waveforms were as shown in Fig. 7. The pressure increase has begun within 1 minute and continued for 2 to 3 minutes. An almost constant pressure was maintained thereafter. The waveforms were relatively similar to the waveform in the case of ADP aggregation. The aggregation waveform obtained by the PAR4-activating peptide was not inhibited by aspirin.

The apparatus in Fig. 8 was prepared for use in the following experiments.

### Example 7

As the container 1 (blood reservoir), an acrylic container having a capacity of 250 µl (inner diameter, 6 mm; depth, 16 mm) was used.

As the filter, a circular nickel micromesh filter having 25 µm × 25 µm square openings (pitch size, 45 µm) and a diameter of 1 mm, placed at the center of the filter section was used.

To the blood reservoir, an ADP reagent (manufactured by MC Medical Inc.) was added to a final concentration of 3 µM, and 240 µl of blood collected in a Terumo blood collection tube (Venoject, containing 3.13% sodium citrate) warmed at 37°C was added thereto. The resulting mixture was mixed in the blood reservoir, and the filter device was connected to the blood reservoir. Thirty seconds, 60 seconds, or 90 seconds after mixing of the blood with the ADP reagent, mineral oil was injected into the blood reservoir at a flow rate of 25 µl/minute for allowing the injection into the filter device.

The back pressure exerted on the mineral oil was continuously monitored for 5 minutes at intervals of 1 second using the pressure sensor.

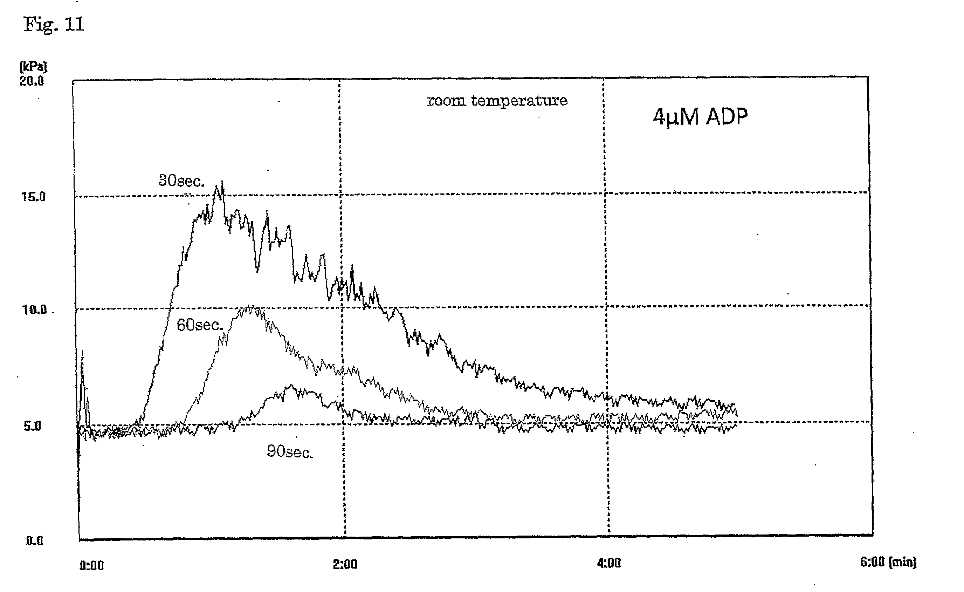

The resulting pressure waveforms were as shown in Fig. 11.

From the results on the pressure pattern, it can be seen that the level of pressure increase observed for the sample which was allowed to pass through the filter 90 seconds after the mixing with the ADP reagent was lower than those observed for the samples which were allowed to pass through the filter 30 seconds or 1 minute after the mixing.

### Example 8

To the blood reservoir, a collagen reagent (Moriya Sangyo K.K.) (final concentration, 4 µg/ml) and blood (240 µl) anticoagulated with sodium citrate were added. After mixing the resulting mixture, the filter device was connected to the blood reservoir. Thirty seconds, 60 seconds, or 90 seconds after mixing of the blood with the collagen reagent, mineral oil was injected into the blood reservoir at a flow rate of 25 µl/minute to inject the blood into the filter device.

The back pressure exerted on the mineral oil was continuously monitored for 5 minutes at intervals of 1 second using the pressure sensor.

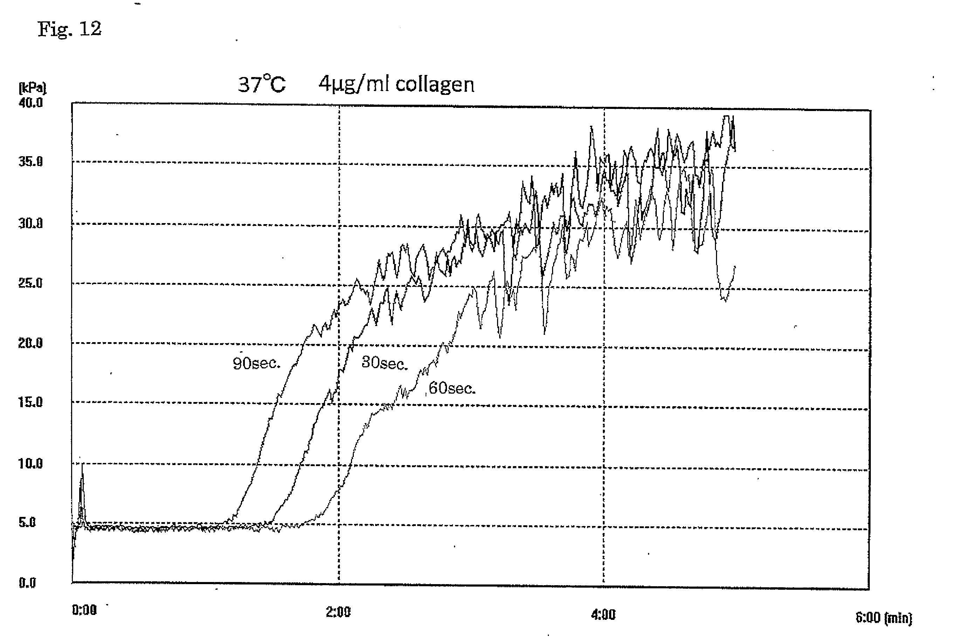

The resulting pressure waveforms were as shown in Fig. 12.

Compared to the blood activated by the ADP reagent, the blood activated by collagen was less influenced by the length of time after the mixing, and showed a steady pressure increase. It can be seen that the influence of the length of time between the mixing and the passing through of the filter varies depending on the platelet-activating reagent.

### DESCRIPTION OF SYMBOLS

10, Platelet aggregability measurement apparatus; 1, Blood storage container; 2, Pump; 3, Pressure sensor; 4, Filter device; 5, Filter; 6, Waste liquid storage section; 7, Air hole; 8, Cap; 9, First end of blood storage container; 11, Second end of blood storage container; 12, Penetrating hole; 13, Blood storage section

## Claims

1. A method for analyzing platelet aggregability and stability of platelet aggregates, said method comprising the steps of:
reacting a platelet-activating reagent with blood in a closed container;
injecting a liquid which is not mixable with blood into said container using a pump connected to a first end of said container, thereby pushing the mixture of the platelet-activating reagent and the blood out from a second end of said container and allowing the mixture to pass through a filter in a filter device connected to the second end of said container; and
measuring the pressure exerted on the pump.

2. The method according to claim 1, wherein the liquid which is not mixable with blood is mineral oil.

3. The method according to claim 1 or 2, wherein said platelet-activating reagent is ADP at a final concentration of 1 to 10 µM, collagen at a final concentration of 0.5 to 10 µg/ml, or arachidonic acid at a final concentration of 0.2 to 20 mM.

4. The method according to any one of claims 1 to 3, wherein the injection rate of the liquid which is not mixable with blood is 5 to 200 µl/minute.

5. The method according to any one of claims 1 to 4, wherein said filter has a mesh having a pitch size or diameter of 10 µm to 50 µm,

6. The method according to any one of claims 1 to 5, wherein said liquid which is not mixable with blood is injected to said container such that said mixture of the platelet-activating reagent and the blood reaches said filter 20 seconds to 2 minutes after the mixing of the platelet-activating reagent with the blood.

7. An apparatus for analyzing platelet aggregability and stability of platelet aggregates, said apparatus comprising a container for storing blood, a pump air-tightly connected to a first end of said container, a sensor for measuring the pressure exerted on said pump, and a filter device air-tightly connected to a second end of said container.

8. The apparatus according to claim 7, wherein the container for storing blood is composed of a blood storage section and a cap for tightly sealing the blood storage section.

9. The apparatus according to claim 8, wherein the container for storing blood is connected to a filter device through said cap.

10. The apparatus according to any one of claims 7 to 9, wherein said filter device comprises a filter section and a waste liquid storage section.

11. The apparatus according to claim 10, wherein an air hole(s) is/are present in said waste liquid storage section.
